# EUROPEAN PATENT APPLICATION

(11) **EP 4 509 084 A1**
(43) Date of publication of application: **19.02.2025**
(21) Application number: 23803617.2
(22) Date of filing: 11.05.2023
(51) Int. Cl.: A61B 34/30

(54) **SURGERY SUPPORT SYSTEM AND METHOD FOR CONTROLLING SURGERY SUPPORT SYSTEM**

(30) Priority: 13.05.2022 JP 2022079492
(71) Applicant: KAWASAKI JUKOGYO KABUSHIKI KAISHA, Kobe-shi, Hyogo 650-8670 (JP)
(72) Inventor: ICHII, Tetsuo, Kobe-shi, Hyogo 650-8670 (JP); TOJO, Tsuyoshi, Kobe-shi, Hyogo 650-8670 (JP)
(74) Representative: Hoffmann Eitle
(86) International application number: PCT/JP2023/017791
(87) International publication number: WO 2023/219143

(57) **Abstract**

In a robotic surgical system (100), a variable control parameter (h) is variable within a predetermined range by operating an operation unit (33b), and a control device (130) is configured or programmed to control a robot main body (1) to transition from a current posture to a first posture obtained after adjustment of the variable control parameter (h), based on an operation on the operation unit (33b).

## Description

### Technical Field

The present disclosure relates to a robotic surgical system and a control method for a robotic surgical system.

### Background Art

Conventionally, a robotic surgical system using a robot including a robot arm to which a surgical instrument is attached is known. For example, Japanese Translation of PCT International Application Publication No. 2017-515521 discloses a teleoperational medical system in which a robot arm is moved to a pre-established position. In Japanese Translation of PCT International Application Publication No. 2017-515521, a touchpad includes a button to move the robot arm to the pre-established position. The pre-established position is a stow position, a draping position, a docking position, etc. The stow position is a position of the robot arm at which a robot is put into a compact state when the robot is put away. The draping position is a position of the robot arm suitable for covering the robot arm with a sterile drape. The docking position is a position of the robot arm suitable for docking the robot arm to a cannula. When an operator operates the button on the touchpad, the robot arm automatically moves from a certain position to a position corresponding to the operated button.

### Prior Art

### Patent Document

Patent Document 1: Japanese Translation of PCT International Application Publication No. 2017-515521

### Summary of the Invention

However, in Japanese Translation of PCT International Application Publication No. 2017-515521, the pre-established position is fixed, and thus the robot arm can only transfer from a certain position to a predetermined position. At this time, the robot arm can only transition to a predetermined posture. For example, depending on the condition of a room in which the robot is stowed, it may be desired to store the robot arm such that the footprint of the robot is minimized, or it may be desired to store the robot arm such that the footprint is not necessarily minimized, but the height of the robot is minimized. In Japanese Translation of PCT International Application Publication No. 2017-515521, the operator cannot adjust the position or posture of the robot after the movement or transition according to the situation.

The present disclosure is intended to solve the above problem. The present disclosure aims to provide a robotic surgical system and a control method for a robotic surgical system each capable of adjusting the posture of a robot main body after transition according to the situation.

In order to attain the aforementioned object, a robotic surgical system according to a first aspect of the present disclosure includes a robot main body to which a surgical instrument is attached, a control device configured or programmed to control the robot main body to transition from a current posture to a first posture, and an operation unit to receive an operation of an operator to adjust the first posture before transition of the robot main body from the current posture to the first posture is completed. The first posture is defined by a plurality of control parameters including a variable control parameter, the variable control parameter is variable within a predetermined range by operating the operation unit, and the control device is configured or programmed to control the robot main body to transition from the current posture to the first posture obtained after adjustment of the variable control parameter, based on an operation on the operation unit.

As described above, the robotic surgical system according to the first aspect of the present disclosure includes the operation unit to receive the operation of the operator to adjust the first posture before transition of the robot main body from the current posture to the first posture is completed, and the control device is configured or programmed to control the robot main body to transition from the current posture to the first posture obtained after the adjustment of the variable control parameter, based on the operation on the operation unit. Accordingly, when the robot main body transitions to the first posture, the first posture after transition can be adjusted before the transition is completed. Therefore, the operator can transition the robot main body to a posture appropriate for the situation.

A control method for a robotic surgical system according to a second aspect of the present disclosure includes receiving an operation of an operator to adjust a variable control parameter, which is variable within a predetermined range, to adjust a first posture before transition of a robot main body to which a surgical instrument is attached from a current posture to the first posture is completed, and transitioning the robot main body from the current posture to the first posture obtained after adjustment of the variable control parameter.

As described above, the control method for the robotic surgical system according to the second aspect of the present disclosure includes receiving the operation of the operator to adjust the variable control parameter, which is variable within the predetermined range, to adjust the first posture before transition of the robot main body from the current posture to the first posture is completed, and transitioning the robot main body from the current posture to the first posture obtained after the adjustment of the variable control parameter. Accordingly, when the robot main body transitions to the first posture, the first posture after transition can be adjusted before the transition is completed. Therefore, it is possible to provide the control method for the robotic surgical system that allows the operator to transition the robot main body to a posture appropriate for the situation.

According to the present disclosure, the operator can transition the robot main body to a posture appropriate to the situation.

### Brief Description of the Drawings

FIG. 1 is a diagram showing the configuration of a robotic surgical system according to an embodiment.
FIG. 2 is a diagram showing a display of a medical cart according to the embodiment.
FIG. 3 is a diagram showing the configuration of the medical cart according to the embodiment.
FIG. 4 is a diagram showing the configuration of a robot arm according to the embodiment.
FIG. 5 is a diagram showing a pair of forceps.
FIG. 6 is a perspective view showing the configuration of an arm operation unit according to the embodiment.
FIG. 7 is a diagram for illustrating translational movement of the robot arm.
FIG. 8 is a diagram for illustrating rotational movement of the robot arm.
FIG. 9 is a control block diagram of a surgical robot according to the embodiment.
FIG. 10 is a control block diagram of the robot arm according to the embodiment.
FIG. 11 is a control block diagram of the medical cart and a positioner according to the embodiment.
FIG. 12 is a diagram for illustrating transition of the surgical robot from a current posture to a first posture.
FIG. 13 is a diagram showing a joystick for adjusting control parameters.
FIG. 14 is a diagram showing the surgical robot in a stored posture when a variable control parameter h is 0.
FIG. 15 is a diagram showing the surgical robot in the stored posture when the variable control parameter h is 0.5.
FIG. 16 is a diagram showing the surgical robot in the stored posture when the variable control parameter h is 1.
FIG. 17 is a diagram showing the surgical robot in a draping posture when the variable control parameter h is 0.
FIG. 18 is a diagram showing the surgical robot in the draping posture when the variable control parameter h is 0.5.
FIG. 19 is a diagram showing the surgical robot in the draping posture when the variable control parameter h is 1.
FIG. 20 is a diagram for illustrating control parameters when the surgical robot transitions from the current posture to the first posture.
FIG. 21 is a diagram showing control parameters for the rotation angles of joints of the robot arm.
FIG. 22 is a diagram showing control parameters for the position coordinates of the positioner and the rotation angles of the joints.
FIG. 23 is a flowchart for illustrating a control method for the surgical robot according to the embodiment.
FIG. 24 is a diagram showing a display of the medical cart according to the embodiment when a surgical site is set.
FIG. 25 is a diagram showing the display of the medical cart according to the embodiment when the medical cart approaches a patient.

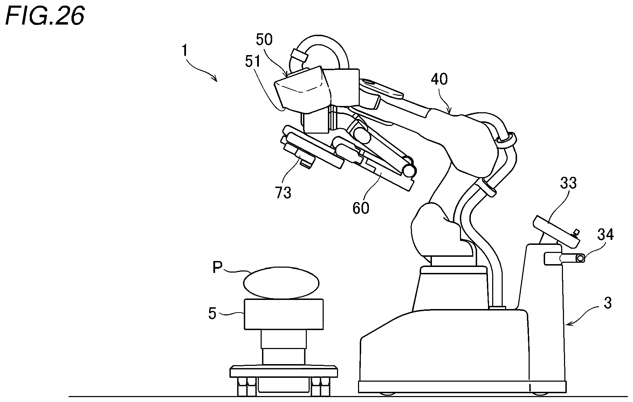

FIG. 26 is a diagram showing a state in which an imager is imaging the patient.
FIG. 27 is a diagram showing a state before alignment between trocars and marks displayed on the display.
FIG. 28 is a diagram showing a state after alignment between the trocars and the marks displayed on the display.
FIG. 29 is a diagram showing an endoscope.
FIG. 30 is a diagram showing a pivot position teaching instrument.

### Modes for Carrying Out the Invention

### Configuration of Robotic Surgical System

The configuration of a robotic surgical system 100 according to this embodiment is now described. The robotic surgical system 100 includes a surgical robot 1 and a remote control apparatus 2.

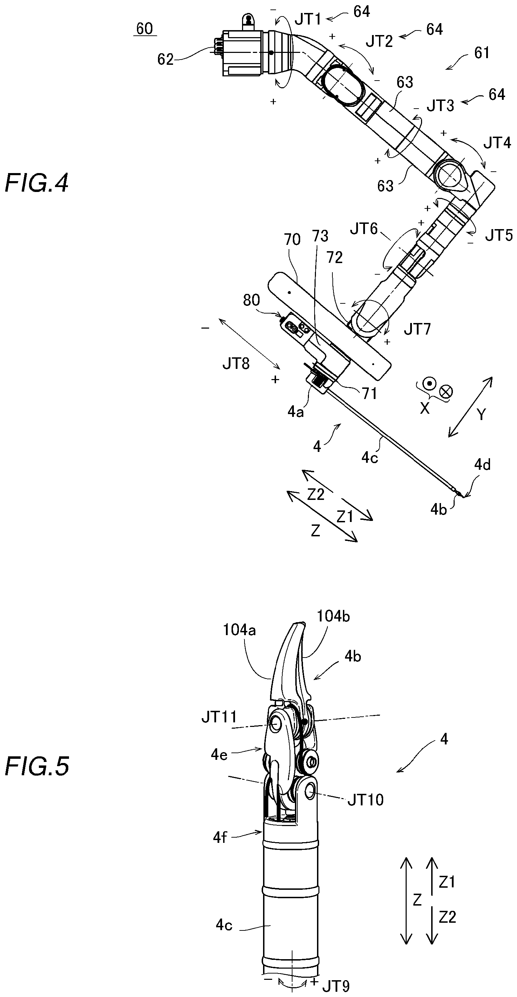

In this specification, the longitudinal direction of a surgical instrument 4 is defined as a Z direction, as shown in FIG. 4. The distal end side of the surgical instrument 4 is defined as a Z1 side, and the proximal end side of the surgical instrument 4 is defined as a Z2 side. A direction perpendicular to the Z direction is defined as an X direction. A direction perpendicular to the Z direction and the X direction is defined as a Y direction.

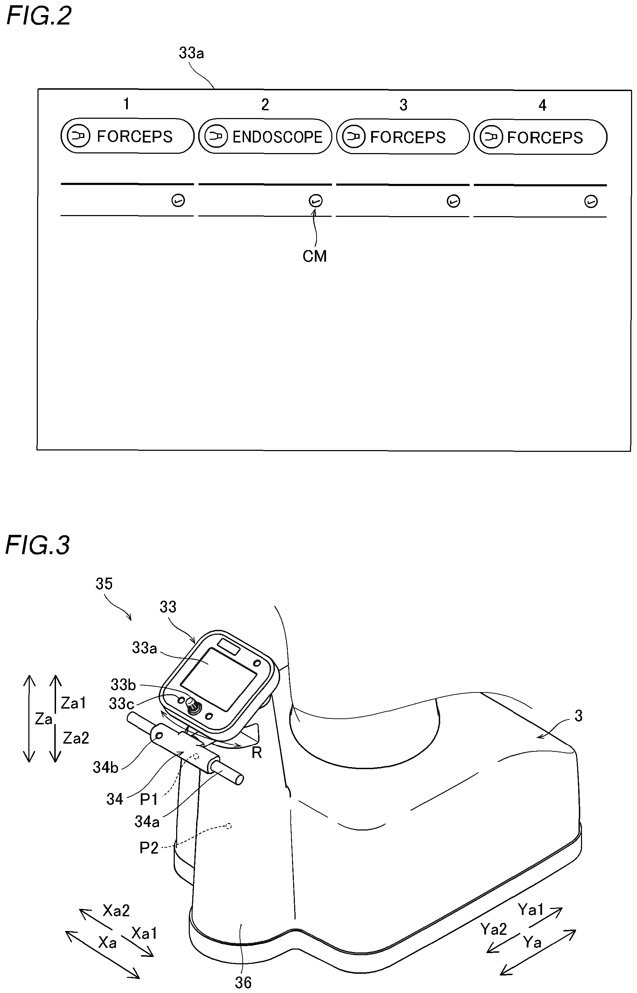

In this specification, as shown in FIG. 3, a right-left direction as viewed by an operator operating a display 33a of an input 33 is defined as an Xa direction. A right direction is defined as an Xa1 direction, and a left direction is defined as an Xa2 direction. A forward-rearward direction as viewed by the operator who operates the display 33a of the input 33 is defined as a Ya direction. A forward direction is defined as a Ya1 direction, and a rearward direction is defined as a Ya2 direction. A direction perpendicular to a floor surface on which the surgical robot 1 is placed is defined as a Za direction. An upward direction is defined as a Za1 direction, and a downward direction is defined as a Za2 direction.

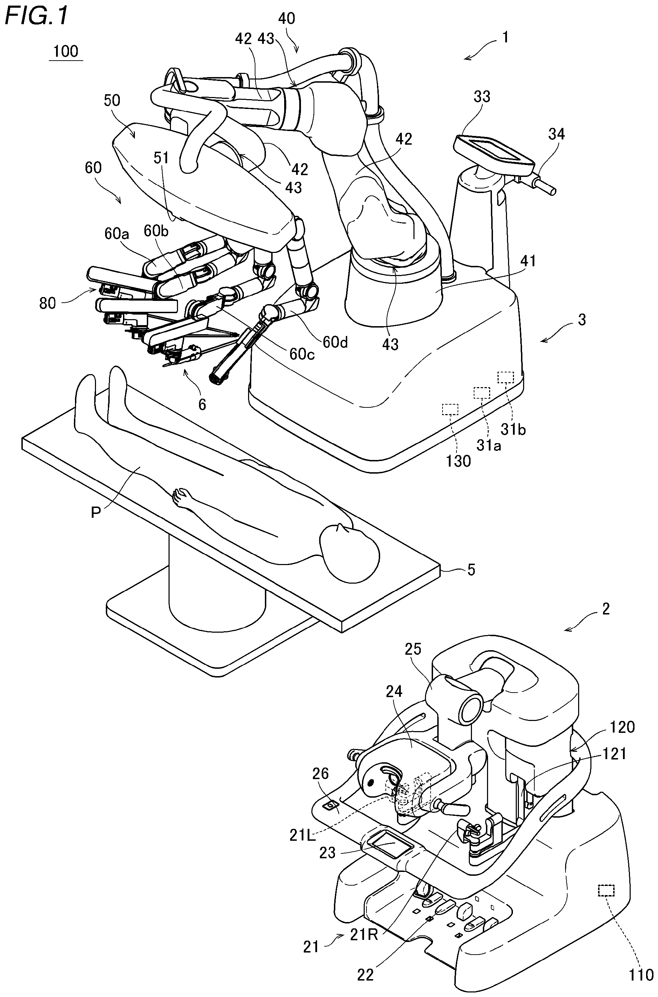

As shown in FIG. 1, the surgical robot 1 is arranged in an operating room. The remote control apparatus 2 is spaced apart from the surgical robot 1. An operator such as a doctor inputs a command to the remote control apparatus 2 to cause the surgical robot 1 to perform a desired operation. The remote control apparatus 2 transmits the input command to the surgical robot 1. The surgical robot 1 operates based on the received command. The surgical robot 1 is arranged in the operating room that is a sterilized sterile field.

### Configuration of Surgical Robot

As shown in FIG. 1, the surgical robot 1 includes a medical cart 3, a positioner 40, an arm base 50, a plurality of robot arms 60, and arm operation units 80. The surgical robot 1 is an example of a robot main body. The positioner 40 is an example of a robot arm mover.

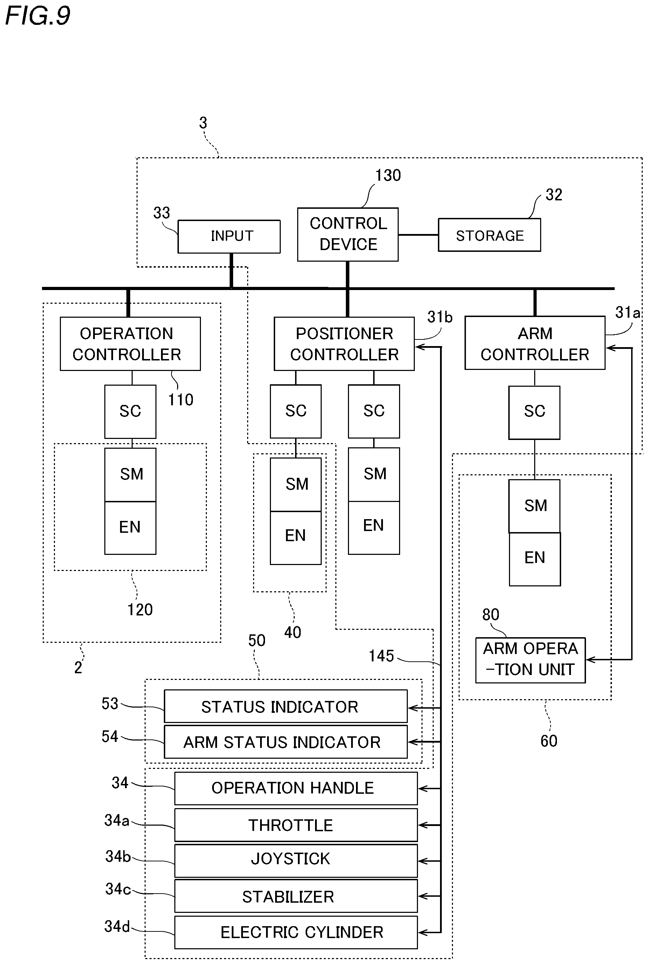

As shown in FIG. 3, the medical cart 3 moves the positioner 40. The medical cart 3 includes the input 33. The input 33 receives operations to move the positioner 40, the arm base 50, and the plurality of robot arms 60 or change their postures mainly in order to prepare for surgery before the surgery. The medical cart 3 includes an operation handle 34, and a stabilizer 34c and an electric cylinder 34d that are shown in FIG. 9.

As shown in FIG. 2, the input 33 includes the display 33a. The display 33a is a liquid crystal panel, for example. Numbers corresponding to the plurality of robot arms 60 are displayed on the display 33a. Furthermore, the type of surgical instrument 4 attached to each of the plurality of robot arms 60 is displayed on the display 33a. A check mark CM indicating that a pivot position PP described below has been taught is displayed on the display 33a.

As shown in FIG. 3, a cart positioner operation unit 35 is supported by a cart positioner operation support 36 at the rear of the medical cart 3, and the medical cart 3 or the positioner 40 is moved by operating the cart positioner operation unit 35. The cart positioner operation unit 35 includes the input 33 and the operation handle 34. The input 33 includes the display 33a, a joystick 33b, and an enable switch 33c. The joystick 33b is arranged in the vicinity of or adjacent to the input 33 of the medical cart 3. The positioner 40 is moved three-dimensionally by selecting an operation mode displayed on the input 33 and operating the joystick 33b. The joystick 33b is an example of an adjuster or an operation unit.

The enable switch 33c is arranged in the vicinity of or adjacent to the joystick 33b of the cart positioner operation unit 35. The enable switch 33c enables or disables movement of the positioner 40. When the joystick 33b is operated while the enable switch 33c is pressed to enable movement of the positioner 40, the positioner 40 is moved.

The operation handle 34 is arranged in the vicinity of the display 33a of the cart positioner operation unit 35. The operation handle 34 includes a throttle 34a that is gripped and twisted by an operator such as a nurse or a technician to operate movement of the medical cart 3. Specifically, the operation handle 34 is arranged below the input 33. As the throttle 34a is twisted from the near side to the far side, the medical cart 3 moves forward. As the throttle 34a is twisted from the far side to the near side, the medical cart 3 moves rearward. The speed of the medical cart 3 is changed according to a twisting amount of the throttle 34a. The operation handle 34 is rotatable to the left and right shown by an R direction, and the medical cart 3 is turned with rotation of the operation handle 34.

An enable switch 34b for enabling or disabling movement of the medical cart 3 is provided on the operation handle 34 of the cart positioner operation unit 35. When the throttle 34a of the operation handle 34 is operated while the enable switch 34b is pressed to enable movement of the medical cart 3, the medical cart 3 is moved.

As shown in FIG. 1, the positioner 40 includes a 7-axis articulated robot, for example. The positioner 40 is arranged on the medical cart 3. The positioner 40 adjusts the position of the arm base 50. The positioner 40 moves the position of the arm base 50 three-dimensionally.

The positioner 40 includes a base 41 and a plurality of links 42 coupled to the base 41. The plurality of links 42 are connected to each other by joints 43.

The arm base 50 is attached to a distal end of the positioner 40. A proximal end of each of the plurality of robot arms 60 is attached to the arm base 50. Each of the plurality of robot arms 60 is able to take a folded and stored posture. The arm base 50 and the plurality of robot arms 60 are covered with sterile drapes and used. Moreover, each of the robot arms 60 supports the surgical instrument 4.

A status indicator 53 and an arm status indicator 54 that are shown in FIG. 9 are provided on the arm base 50. The status indicator 53 indicates the status of the robotic surgical system 100. The arm status indicator 54 indicates the statuses of the robot arms 60.

The plurality of robot arms 60 are arranged. Specifically, four robot arms 60a, 60b, 60c, and 60d are arranged. The robot arms 60a, 60b, 60c, and 60d have the same or similar configurations as each other.

As shown in FIG. 4, each robot arm 60 includes an arm portion 61, a first link 72, a second link 73, and a translation mechanism 70. The robot arm 60 includes a JT1 axis, a JT2 axis, a JT3 axis, a JT4 axis, a JT5 axis, a JT6 axis, and a JT7 axis as rotation axes and a JT8 axis as a linear motion axis. The JT1 to JT7 axes are rotation axes of joints 64 of the arm portion 61. Furthermore, the JT7 axis is a rotation axis of the first link 72. The JT8 axis is a linear motion axis along which the translation mechanism 70 moves the second link 73 relative to the first link 72 along the Z direction. The arm portion 61 includes a base 62, links 63, and the joints 64.

The arm portion 61 includes a 7-axis articulated robot arm. The first link 72 is arranged at a distal end of the arm portion 61. An arm operation unit 80 described below is attached to the second link 73. The translation mechanism 70 is arranged between the first link 72 and the second link 73. A holder 71 that holds the surgical instrument 4 is arranged on the second link 73.

The surgical instrument 4 is attached to a distal end of each of the plurality of robot arms 60. The surgical instrument 4 includes a replaceable instrument, an endoscope 6 to capture an image of a surgical site, or a pivot position teaching instrument 7 to teach the pivot position PP described below, for example. The surgical instrument 4 as the instrument includes a driven unit 4a, a pair of forceps 4b, and a shaft 4c.

As shown in FIG. 1, the endoscope 6 is attached to the distal end of one of the plurality of robot arms 60, such as the robot arm 60c, and surgical instruments 4 other than the endoscope 6 are attached to the distal ends of the remaining robot arms 60a, 60b, and 60d, for example. The endoscope 6 is attached to one of two robot arms 60b and 60c arranged in the center among the four robot arms 60 arranged adjacent to each other.

### Configuration of Instrument

As shown in FIG. 5, the pair of forceps 4b is provided at a distal end of the instrument, for example. At the distal end of the instrument, in addition to the pair of forceps 4b, a pair of scissors, a grasper, a needle holder, a microdissector, a stable applier, a tacker, a suction cleaning tool, a snare wire, a clip applier, etc. are arranged as instruments having joints. At the distal end of the instrument, a cutting blade, a cautery probe, a washer, a catheter, a suction orifice, etc. are arranged as instruments having no joint.

The pair of forceps 4b includes a first support 4e and a second support 4f. The first support 4e supports the proximal end sides of jaw members 104a and 104b such that the proximal end sides of the jaw members 104a and 104b are rotatable about a JT11 axis. The second support 4f supports the proximal end side of the first support 4e such that the proximal end side of the first support 4e is rotatable about a JT10 axis. The shaft 4c rotates about a JT9 axis. The jaw members 104a and 104b pivot about the JT11 axis to open and close.

### Configuration of Arm Operation Unit

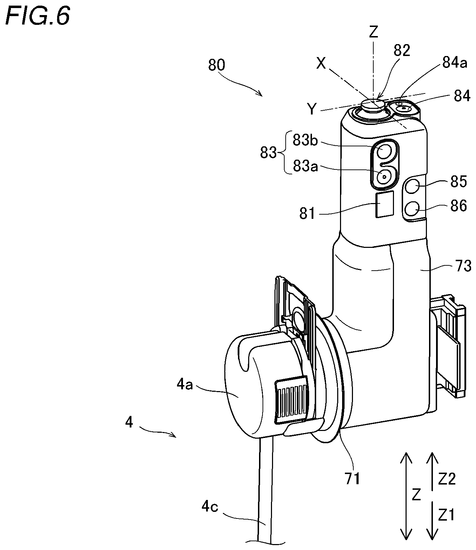

As shown in FIG. 6, the arm operation unit 80 is attached to the robot arm 60 to operate the robot arm 60. Specifically, the arm operation unit 80 is attached to the second link 73.

The arm operation unit 80 includes an enable switch 81, a joystick 82, and linear switches 83, a mode switching button 84, a mode indicator 84a, a pivot button 85, and an adjustment button 86.

The enable switch 81 enables or disables movement of the robot arm 60 in response to the joystick 82 and the linear switches 83. When the enable switch 81 is pressed by an operator such as a nurse or an assistant grasping the arm operation unit 80, movement of the surgical instrument 4 by the robot arm 60 is enabled.

The joystick 82 is an operation tool to control movement of the surgical instrument 4 by the robot arm 60. The joystick 82 controls a moving direction and a moving speed of the robot arm 60. The robot arm 60 is moved in accordance with a tilting direction and a tilting angle of the joystick 82.

The linear switches 83 are switches to move the surgical instrument 4 in the Z direction, which is the longitudinal direction of the surgical instrument 4. The linear switches 83 include a linear switch 83a to move the surgical instrument 4 in a direction in which the surgical instrument 4 is inserted into a patient P, and a linear switch 83b to move the surgical instrument 4 in a direction in which the surgical instrument 4 is moved away from the patient P. Both the linear switch 83a and the linear switch 83b are push-button switches.

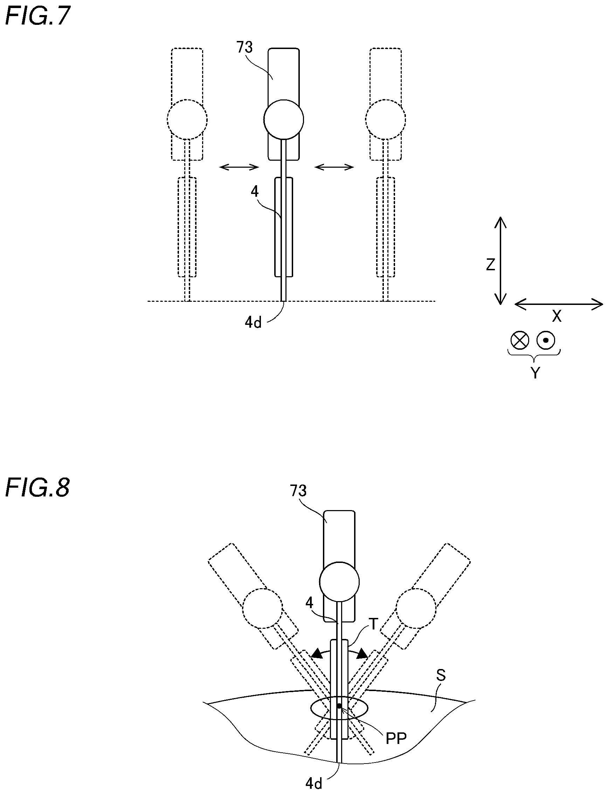

The mode switching button 84 is a push-button switch to switch between a mode for translationally moving the surgical instrument 4 as shown in FIG. 7 and a mode for rotationally moving the surgical instrument 4 as shown in FIG. 8. As shown in FIG. 7, in the mode for translationally moving the robot arm 60, the robot arm 60 is moved such that a distal end 4d of the surgical instrument 4 is moved in an X-Y plane. As shown in FIG. 8, in the mode for rotationally moving the robot arm 60, the robot arm 60 is moved such that the surgical instrument 4 is rotationally moved about a center of the JT11 axis of the pair of forceps 4b as a fulcrum when any pivot position PP is not stored in a storage 32, and the surgical instrument 4 is rotationally moved about the pivot position PP as a fulcrum when the pivot position PP is stored in the storage 32. In this case, the surgical instrument 4 is rotationally moved with the shaft 4c of the surgical instrument 4 inserted into a trocar T. The mode switching button 84 is arranged on a Z-direction side surface of the arm operation unit 80.

The mode indicator 84a indicates a switched mode. The mode indicator 84a is on to indicate a rotational movement mode and is off to indicate a translational movement mode. Furthermore, the mode indicator 84a also serves as a pivot position indicator that indicates that the pivot position PP has been taught. The mode indicator 84a is arranged on the Z-direction side surface of the arm operation unit 80.

The pivot button 85 is a push-button switch to teach the pivot position PP that serves as a fulcrum for movement of the surgical instrument 4 attached to the robot arm 60.

The adjustment button 86 is a button to optimize the position of the robot arm 60. After the pivot position PP for the robot arm 60 to which the endoscope 6 has been attached is taught, the positions of the other robot arms 60 and the arm base 50 are optimized when the adjustment button 86 is pressed.

### Remote Control Apparatus

As shown in FIG. 1, the remote control apparatus 2 is arranged inside or outside the operating room, for example. The remote control apparatus 2 includes operation units 120 including arms 121 and operation handles 21, foot pedals 22, a touch panel 23, a monitor 24, a support arm 25, and a support bar 26. The operation units 120 includes operation handles for the operator such as a doctor to input a command.

The operation units 120 each include a handle to operate the surgical instrument 4. The operation units 120 each receive an operation amount for the surgical instrument 4. The operation units 120 include an operation unit 120 that is located on the left side as viewed from the operator such as a doctor and is to be operated by the left hand of the operator, and an operation unit 120 that is located on the right side and is to be operated by the right hand of the operator. The operation unit 120L and the operation unit 120R include an operation handle 21L and an operation handle 21R, respectively.

The monitor 24 is a scope-type display that displays an image captured by the endoscope 6. The support arm 25 supports the monitor 24 so as to align the height of the monitor 24 with the height of the face of the operator such as a doctor. The touch panel 23 is arranged on the support bar 26. The head of the operator is detected by a sensor provided in the vicinity of the monitor 24 such that the surgical robot 1 can be operated by the remote control apparatus 2. The operator operates the operation units 120 and the foot pedals 22 while visually recognizing an affected area on the monitor 24. Thus, a command is input to the remote control apparatus 2. The command input to the remote control apparatus 2 is transmitted to the surgical robot 1.

### Configuration of Control System

As shown in FIG. 9, the robotic surgical system 100 includes a control device 130, an arm controller 31a, a positioner controller 31b, and operation controllers 110.

The control device 130 is accommodated in the medical cart 3 to communicate with the arm controller 31a and the positioner controller 31b, and controls the entire robotic surgical system 100. Specifically, the control device 130 communicates with and controls the arm controller 31a, the positioner controller 31b, and the operation controllers 110. The control device 130 is connected to the arm controller 31a, the positioner controller 31b, and the operation controllers 110 through a LAN, for example. The control device 130 is arranged inside the medical cart 3.

The arm controller 31a is arranged for each of the plurality of robot arms 60. That is, the same number of arm controllers 31a as the plurality of robot arms 60 are arranged inside the medical cart 3.

As shown in FIG. 9, the input 33 is connected to the control device 130 through a LAN, for example. The status indicator 53, the arm status indicator 54, the operation handle 34, the throttle 34a, the joystick 33b, the stabilizer 34c, and the electric cylinder 34d establish a serial communication connection with the positioner controller 31b through a wire line 145 by means of a communication network that allows information to be shared with each other. Although FIG. 9 shows that the status indicator 53, the arm status indicator 54, etc. are all connected to one wire line 145, in reality, the wire line 145 is arranged for each of the status indicator 53, the arm status indicator 54, the operation handle 34, the throttle 34a, the joystick 33b, the stabilizer 34c, and the electric cylinder 34d.

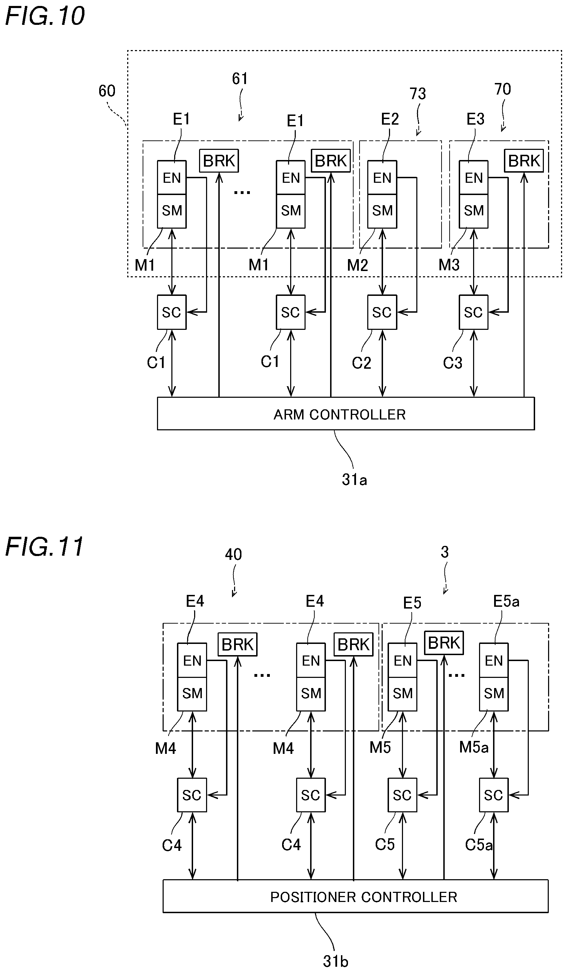

As shown in FIG. 10, the arm portion 61 includes a plurality of servomotors M1, encoders E1, and speed reducers so as to correspond to a plurality of joints 64. The encoders E1 detect rotation angles of the servomotors M1. The speed reducers slow down rotation of the servomotors M1 to increase the torques. Inside the medical cart 3, servo controllers C1 that control the servomotors M1 are provided adjacent to the arm controller 31a. The encoders E1 that detect the rotation angles of the servomotors M1 are electrically connected to the servo controllers C1.

The joints 64 of the arm portion 61 and the joints 43 of the positioner 40 include brakes BRK. Furthermore, the front wheels of the medical cart 3, the arm base 50, and the translation mechanism 70 include brakes BRK. The arm controller 31a unidirectionally transmits control signals to the brakes BRK of the joints 64 of the arm portion 61, and the translation mechanism 70. The control signals are signals for turning on/off the brakes BRK. The signals for turning on the brakes BRK include signals for maintaining the brakes BRK in an enabled state. The same applies to control signals from the positioner controller 31b to the brakes BRK of the joints 43 of the positioner 40 and the arm base 50. On startup, all the brakes BRK of the arm base 50, the arm portion 61, and the translation mechanism 70 are turned off but the servomotors SM are driven against gravity to maintain the postures of the robot arm 60 and the arm base 50. When an error occurs in the robotic surgical system 100, the brakes BRK of the arm base 50, the arm portion 61 and the translation mechanism 70 are turned on. When the error in the robotic surgical system 100 is reset, the brakes BRK of the arm base 50, the arm portion 61, and the translation mechanism 70 are turned off. When a shutdown operation is performed in the robotic surgical system 100, the brakes BRK of the arm base 50, the arm portion 61, and the translation mechanism 70 are turned on. The brakes BRK of the front wheels of the medical cart 3 are constantly turned on, and the brakes BRK are deactivated only while the enable switch 34b of the medical cart 3 is being pressed. The brakes BRK of the joints 43 of the positioner 40 are constantly turned on, and the brakes BRK are deactivated only while the enable switch 33c of the medical cart 3 is being pressed.

Servomotors M2 that rotate driven members provided in the driven unit 4a of the surgical instrument 4, encoders E2, and speed reducers are arranged in the second link 73. The encoders E2 detect rotation angles of the servomotors M2. The speed reducers slow down rotation of the servomotors M2 to increase the torques. In the medical cart 3, servo controllers C2 are provided to control the servomotors M2 to drive the surgical instrument 4. The encoders E2 that detect the rotation angles of the servomotors M2 are electrically connected to the servo controllers C2. A plurality of servomotors M2, a plurality of encoders E2, and a plurality of servo controllers C2 are arranged.

The translation mechanism 70 includes a servomotor M3 to translationally move the surgical instrument 4, an encoder E3, and a speed reducer. The encoder E3 detects a rotation angle of the servomotor M3. The speed reducer slows down rotation of the servomotor M3 to increase the torque. In the medical cart 3, a servo controller C3 is provided to control the servomotor M3 to translationally move the surgical instrument 4. The encoder E3 that detects the rotation angle of the servomotor M3 is electrically connected to the servo controller C3.

As shown in FIG. 11, a plurality of servomotors M4, a plurality of encoders E4, and a plurality of speed reducers are provided in the positioner 40 so as to correspond to the plurality of joints 43 of the positioner 40. The encoders E4 detect rotation angles of the servomotors M4. The speed reducers slow down rotation of the servomotors M4 to increase the torques.

The medical cart 3 includes wheels including front wheels as drive wheels and rear wheels that are steered by the operation handle 34. The rear wheels are arranged closer to the operation handle 34 than the front wheels. The medical cart 3 includes servomotors M5 to drive a plurality of front wheels of the medical cart 3, encoders E5, speed reducers, and brakes. The speed reducers slow down rotation of the servomotors M5 to increase the torques. A potentiometer P1 shown in FIG. 3 is provided on the operation handle 34 of the medical cart 3, and the servomotors M5 of the front wheels are driven based on a rotation angle detected by the potentiometer P1 according to the twist of the throttle 34a. Rear wheels of the medical cart 3 are of the dual wheel type, and the rear wheels are steered based on rightward-leftward rotation of the operation handle 34. Furthermore, a potentiometer P2 shown in FIG. 3 is provided on a rotation axis of the operation handle 34 of the medical cart 3, and servomotors M5a, encoders E5a, and speed reducers are provided on the rear wheels of the medical cart 3. The speed reducers slow down rotation of the servomotors M5a to increase the torques. The servomotors M5a are driven based on a rotation angle detected by the potentiometer P2 according to rightward-leftward rotation of the operation handle 34. That is, steering of the rear wheels by the rightward-leftward rotation of the operation handle 34 is power-assisted by the servomotors M5a.

The front wheels of the medical cart 3 are driven such that the medical cart moves in the forward-rearward direction. Furthermore, the operation handle 34 of the medical cart 3 is rotated such that the rear wheels are steered, and the medical cart 3 turns in the right-left direction.

As shown in FIG. 11, in the medical cart 3, servo controllers C4 are provided to control the servomotors M4 to move the positioner 40. The encoders E4 that detect the rotation angles of the servomotors M4 are electrically connected to the servo controllers C4. In the medical cart 3, servo controllers C5 are provided to control the servomotors M5 to drive the front wheels of the medical cart 3. The encoders E5 that detect the rotation angles of the servomotors M5 are electrically connected to the servo controllers C5. In the medical cart 3, servo controllers C5a are provided to control the servomotors M5a to power-assist steering of the rear wheels of the medical cart 3. The encoders E5a that detect the rotation angles of the servomotors M5a are electrically connected to the servo controllers C5a.

As shown in FIG. 9, the control device 130 controls the robot arm 60 based on an operation received by the arm operation unit 80. For example, the control device 130 controls the robot arm 60 based on an operation received by the joystick 82 of the arm operation unit 80. Specifically, the arm controller 31a outputs an input signal input from the joystick 82 to the control device 130. The control device 130 generates position commands based on the received input signal and the rotation angles detected by the encoders N1, and outputs the position commands to the servo controllers C1 via the arm controller 31a. The servo controllers C1 generate current commands based on the position commands input from the arm controller 31a and the rotation angles detected by the encoders E1, and output the current commands to the servomotors M1. Thus, the robot arm 60 is moved according to an operation command input to the joystick 82.

The control device 130 controls the robot arm 60 based on an input signal from either linear switch 83 of the arm operation unit 80. Specifically, the arm controller 31a outputs the input signal input from the linear switch 83 to the control device 130. The control device 130 generates a position command(s) based on the received input signal and the rotation angle(s) detected by the encoders E1 or the encoder E3, and outputs the position command(s) to the servo controllers C1 or the servo controller C3 via the arm controller 31a. The servo controllers C1 or the servo controller C3 generates a current command(s) based on the position command(s) input from the arm controller 31a and the rotation angle(s) detected by the encoders E1 or the encoder E3, and outputs the current command(s) to the servomotors M1 or the servomotor M3. Thus, the robot arm 60 is moved according to an operation command input to the linear switch 83.

The positioner controller 31b is arranged in the medical cart 3. The positioner controller 31b controls the positioner 40 and the medical cart 3. The servomotors SM, the encoders EN, and the speed reducers are provided in the positioner 40 so as to correspond to the plurality of joints 43 of the positioner 40. The servo controllers SC are provided in the medical cart 3 to control the servomotors SM of the positioner 40. The servomotors SM that drive the plurality of front wheels of the medical cart 3, the encoders EN, the speed reducers, the servo controllers SC, and the brakes are provided in the medical cart 3.

The operation controllers 110 are arranged in a main body of the remote control apparatus 2. The operation controllers 110 control the operation units 120. The operation controllers 110 are provided so as to correspond to the left-handed operation unit 120 and the right-handed operation unit 120, respectively. Servomotors SM, encoders EN, and speed reducers are provided in the operation units 120 so as to correspond to a plurality of joints of the operation units 120. Servo controllers SC that control the servomotors SM of the operation units 120 are provided adjacent to the operation controllers 110 in the main body of the remote control apparatus 2.

### Transition of Posture of Surgical Robot

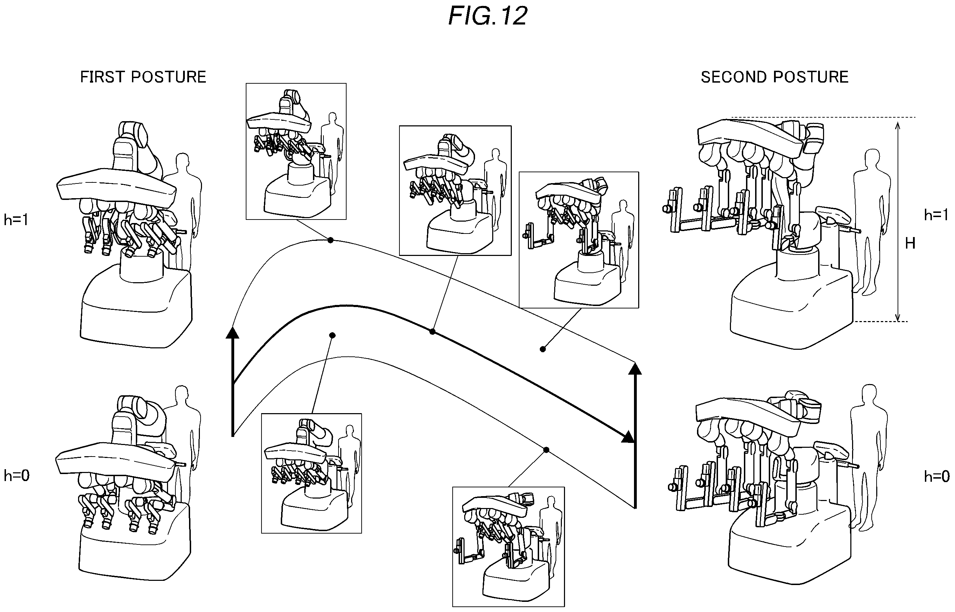

In this embodiment, as shown in FIG. 12, the control device 130 controls the robot main body to transition from a current posture to a first posture. The first posture is defined by a plurality of control parameters including a variable control parameter h described below. The variable control parameter h can be changed within a predetermined range by operating the joystick 33b. The plurality of control parameters also includes a fixed control parameter. The first posture includes at least one of a storage posture, a draping posture, a standby posture, a roll-in posture, a cleaning posture, or a transportation posture. In this embodiment, the first posture includes all of the above postures. The storage posture refers to a posture in which the surgical robot 1 is stored. The draping posture refers to a posture in which the surgical robot 1 is covered with a sterile drape. The standby posture refers to a posture in which the surgical robot 1 is kept waiting before the start of surgery. The roll-in posture refers to a posture in which the surgical robot 1 is moved to the patient P. The cleaning posture refers to a posture in which the surgical robot 1 is cleaned. The transportation posture refers to a posture in which the surgical robot 1 is transported.

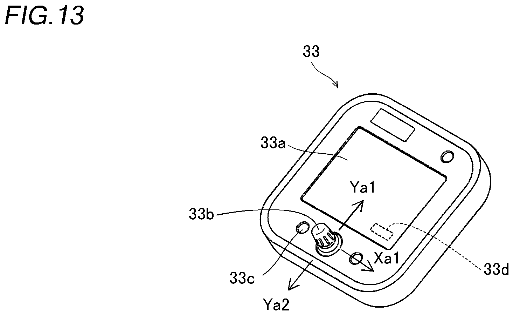

As shown in FIG. 13, buttons 33d for transitioning to the above first postures are displayed on the display 33a of the input 33. When the operator such as a nurse or a technician presses any of the buttons 33d on the display 33a, the control device 130 controls the robot main body to transition to the first posture corresponding to the pressed button 33d. Specifically, after the operator presses the button 33d on the display 33a, the joystick 33b is tilted in the Xa1 direction while the enable switch 33c is pressed such that the surgical robot 1 transitions from the current posture to the first posture. The Xa1 direction is an example of a second direction.

In this embodiment, the joystick 33b receives an operation of the operator to adjust the variable control parameter h for adjusting the first posture before the transition of the surgical robot 1 from the current posture to the first posture is completed. The adjustment of the first posture includes an adjustment of at least one of the height H of the surgical robot 1 in the first posture, the amount of forward protrusion of the surgical robot 1 in the first posture, the amount of right-left protrusion of the surgical robot 1 in the first posture, or the right-left rotation angle of the surgical robot 1 in the first posture. In this embodiment, the adjustment of the first posture includes all of the above adjustments.

The height H of the surgical robot 1 refers to the height H of the surgical robot 1 in the Za direction. The amount of forward protrusion of the surgical robot 1 refers to the amount of protrusion of the surgical robot 1 in the Ya1 direction. The amount of right-left protrusion of the surgical robot 1 refers to the amount of protrusion of the surgical robot 1 in the Xa1 direction or the Xa2 direction. The right-left rotation angle of the surgical robot 1 refers to the rotation angle of the surgical robot 1 about an axis along the Za direction.

In this embodiment, the control device 130 controls the robot arms 60 and the positioner 40 to transition from the current posture to the first posture obtained after adjustment of the variable control parameter h. That is, the postures of both the robot arms 60 and the positioner 40 are adjusted.

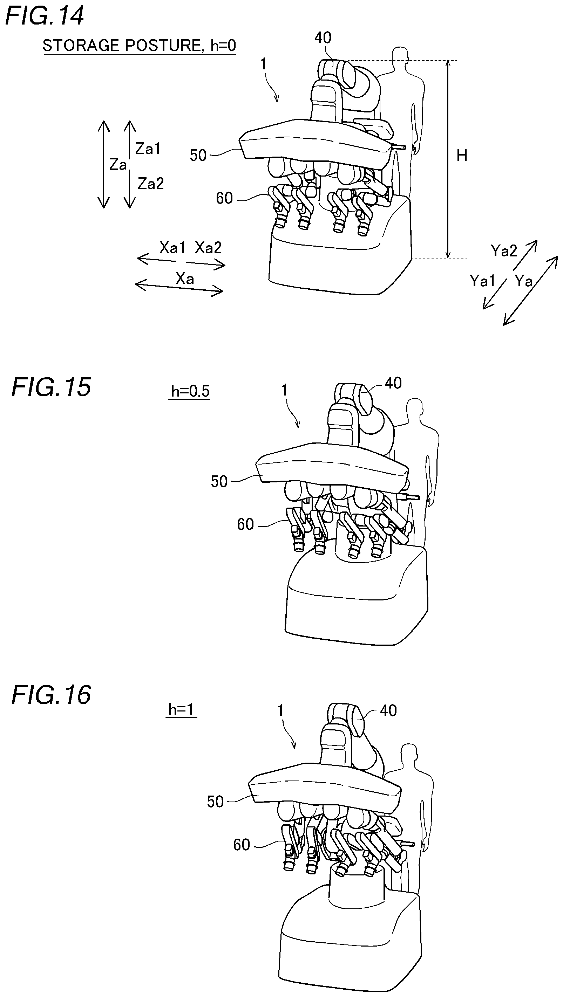

FIGS. 14, 15, and 16 show the storage posture of the surgical robot 1. The height H of the surgical robot 1 increases in the order of FIGS. 14, 15, and 16. FIG. 14 shows the surgical robot 1 in a state in which the height H has not been adjusted. FIGS. 15 and 16 show the surgical robot 1 in a state in which the height H has been adjusted. In FIG. 14, the height H of the surgical robot 1 decreases, while the amount of forward-rearward protrusion of the surgical robot 1 increases. In FIG. 16, the height H of the surgical robot 1 increases, while the amount of forward-rearward protrusion of the surgical robot 1 decreases.

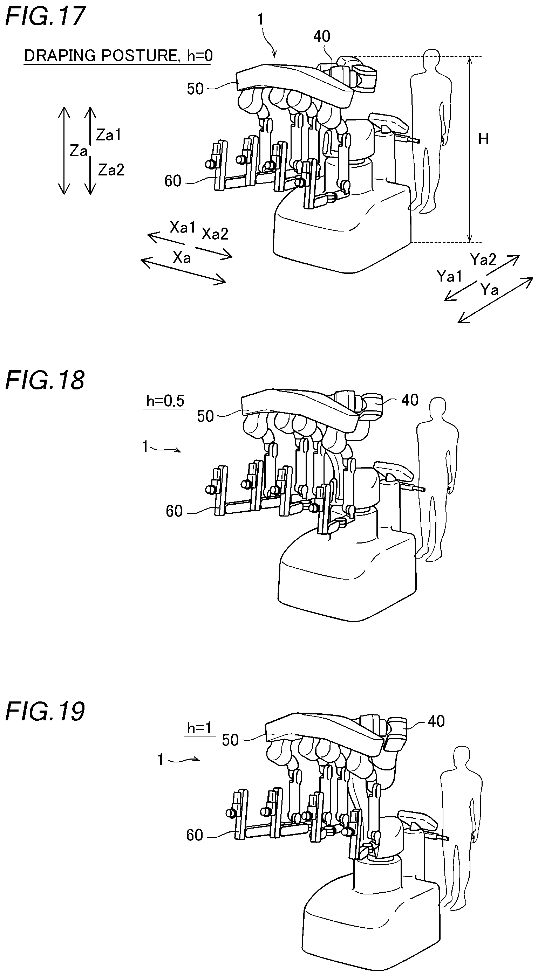

FIGS. 17, 18, and 19 show the draping posture of the surgical robot 1. The height H of the surgical robot 1 increases in the order of FIGS. 17, 18, and 19. FIG. 17 shows the surgical robot 1 in a state in which the height H has not been adjusted. FIGS. 18 and 19 show the surgical robot 1 in a state in which the height H has been adjusted. In FIG. 17, the height H of the surgical robot 1 is low, and thus the operator can easily cover the arm base 50 with a sterile drape. In FIG. 19, the height H of the surgical robot 1 is high, and a distance between the adjacent robot arms 60 is large. Thus, the operator can easily cover the robot arm 60 with a sterile drape. Of the four robot arms 60 arranged on the arm base 50, the central robot arm 60b and the central robot arm 60c are fixed to the arm base 50. The robot arms 60a and 60d arranged at both ends can move linearly, and the distance between the robot arms 60 can be adjusted. The arm base 50 includes a servomotor SM, an encoder EN, a speed reducer, a servo controller SC, and a brake BRK, and the driving force of the servomotor SM can be transmitted to a belt and a pulley to adjust the distance between the robot arms 60. The servo controller SC is arranged in the medical cart 3.

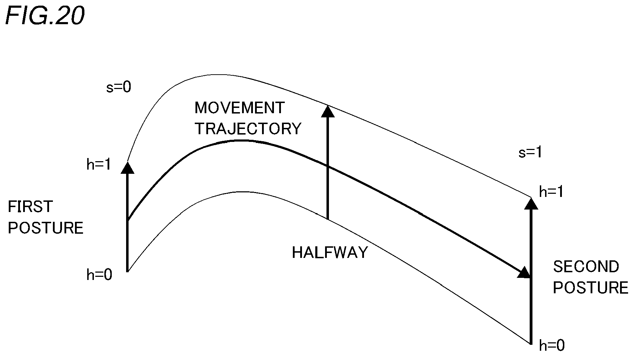

In this embodiment, as shown in FIG. 20, when the joystick 33b receives an operation to adjust the first posture, the control device 130 controls the surgical robot 1 to transition from the current posture to the first posture obtained after adjustment of the variable control parameter h. In FIG. 20, h indicates a control parameter for adjusting the first posture, h = 0 indicates that the first posture has not been adjusted, s indicates the degree of progress of transition of the posture of the surgical robot 1, s = 0 indicates a time point before transition of the surgical robot 1, and s = 1 indicates a time point of completion of transition of the surgical robot 1. In other words, the posture of the surgical robot 1 at the time point of s = 0 is the current posture. The posture of the surgical robot 1 at the time point of s = 1 is the first posture.

In this embodiment, the joystick 33b receives an operation of the operator to adjust the variable control parameter h at any time point from the start of transition of the surgical robot 1 from the current posture to the first posture to the completion of the transition. That is, the joystick 33b receives an operation of the operator to adjust the variable control parameter h at any time point from s = 0 to s = 1. In other words, the joystick 33b receives an operation to adjust the variable control parameter h even halfway between s = 0 and s = 1. As shown in FIG. 12, the intermediate posture of the surgical robot 1 during transition from the current posture to the first posture is determined by the variable control parameter h and the progress degree s.

In this embodiment, the joystick 33b receives an operation of the operator to adjust the variable control parameter h at the start of transition of the surgical robot 1 from the current posture to the first posture. That is, the joystick 33b receives an operation to adjust the current posture at the time point of s = 0. After the current posture is adjusted, the surgical robot 1 is transitioned to the first posture.

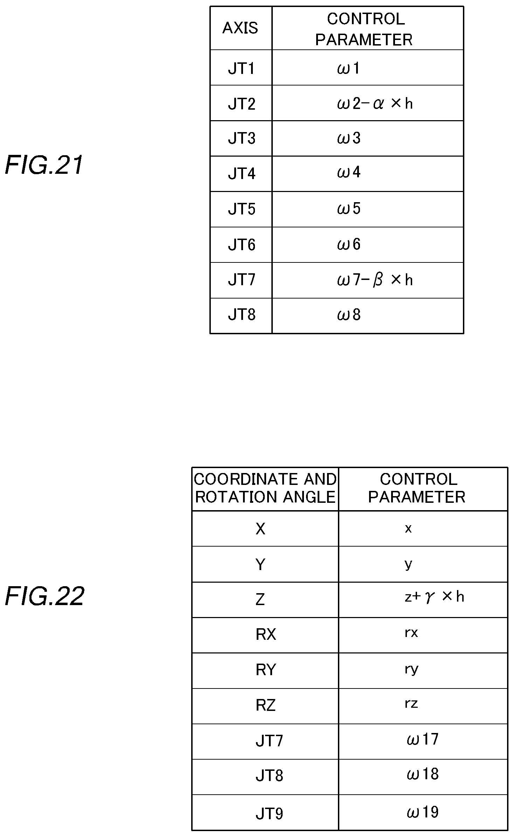

In this embodiment, as shown in FIGS. 21 and 22, the variable control parameter h is included in the plurality of control parameters that define the posture of the surgical robot 1. The joystick 33b adjusts the variable control parameter h included in the plurality of control parameters that define the posture of the surgical robot 1 based on the operation of the operator. Specifically, in this embodiment, the control device 130 performs a control to change at least one of the rotation angles of the joints 43 of the positioner 40 and the joints 64 of the robot arm 60 corresponding to control parameters or the coordinates of the surgical robot 1 corresponding to control parameters based on the adjustment operation received by the joystick 33b. As shown in FIG. 21, for example, the rotation angles of the servomotors SM corresponding to the JT1 to JT8 axes of the robot arm 60 are specified for each first posture. In FIG. 21, ω1 to ω8 are fixed control parameters. The variable control parameter h can be changed within the predetermined range. For example, the upper limit of the variable control parameter h is 1 and the lower limit thereof is 0. The joystick 33b receives adjustment of the variable control parameter h between the upper limit and lower limit of the variable control parameter h. Furthermore, α and β are constants. That is, in an example shown in FIG. 21, the rotation angles of the joints 64 of the robot arm 60 corresponding to the JT2 and JT7 axes are changed based on the adjustment operation received by the joystick 33b. Thus, the first posture of the robot arm 60 is adjusted based on the adjustment operation received by the joystick 33b.

In an example shown in FIG. 22, the coordinates and rotation angle of the positioner 40 are specified for each first posture. RX is the rotation angle about an X-axis, RY is the rotation angle about a Y-axis, and RZ is the rotation angle about the Z-axis. J7 is the rotation axis of the distal end of the positioner 40 that supports the arm base 50. J8 and J9 are the rotation axes of the robot arm 60 with respect to the arm base 50. In FIG. 22, x, y, z, rx, ry, rz, ω17, ω18, and ω19 are fixed control parameters. In the example shown in FIG. 22, the Z-axis coordinate includes h, which is a control parameter having an upper limit and a lower limit. Note that γ is a constant. That is, in the example shown in FIG. 22, the position of the positioner 40 in a Z-axis direction is changed based on the adjustment operation received by the joystick 33b. Thus, the height H of the surgical robot 1 in the first posture is adjusted based on the adjustment operation received by the joystick 33b.

In this embodiment, as shown in FIG. 13, when the joystick 33b is operated by the operator, an operation is received to adjust the variable control parameter h. Specifically, when the joystick 33b is tilted in the Ya direction by the operator, an operation is received to adjust the variable control parameter h. When the joystick 33b is tilted in the Xa1 direction intersecting with the Ya direction by the operator, the control device 130 controls the surgical robot 1 to transition from the current posture to the first posture obtained after adjustment of the variable control parameter h. For example, when the joystick 33b is tilted in the Ya1 direction, the value of the variable control parameter h increases. When the joystick 33b is tilted in the Ya2 direction, the value of the variable control parameter h decreases. When the joystick 33b is tilted in the Xa1 direction, the value of the progress degree s increases. The Ya direction is an example of a first direction.

In this embodiment, the joystick 33b receives an operation to adjust the first posture according to the magnitude of the tilt in the Ya direction. That is, as the tilt of the joystick 33b in the Ya1 direction increases, the value of the variable control parameter h increases faster. As the tilt of the joystick 33b in the Ya2 direction increases, the value of the variable control parameter h decreases faster. As the tilt of the joystick 33b in the Xa1 direction increases, the value of the progress degree s increases faster.

In this embodiment, when the operator tilts the joystick 33b in the Ya direction while transition of the posture of the surgical robot 1 is enabled by the enable switch 33c, an operation is received to adjust the variable control parameter h. When the operator tilts the joystick 33b in the Xa1 direction while transition of the posture of the surgical robot 1 is enabled by the enable switch 33c, the control device 130 controls the surgical robot 1 to transition from the current posture to the first posture. That is, while the enable switch 33c is pressed by the operator, an operation on the joystick 33b is received.

### Control Method for Robotic Surgical System

A control method for the robotic surgical system 100 is now described. Four trocars T4 are arranged in advance on the body surface S of the patient P placed on a surgical table 5. The surgical instrument 4 is not attached to each of the robot arms 60. An example is described below in which adjustment of the variable control parameter h is received by the joystick 33b when the surgical robot 1 transitions to the roll-in posture.

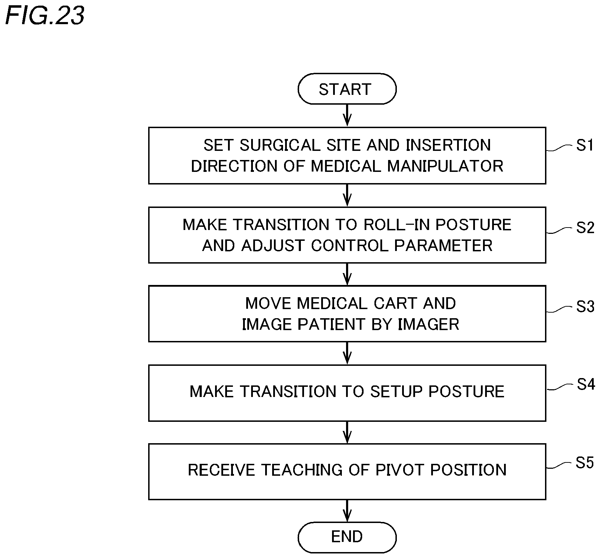

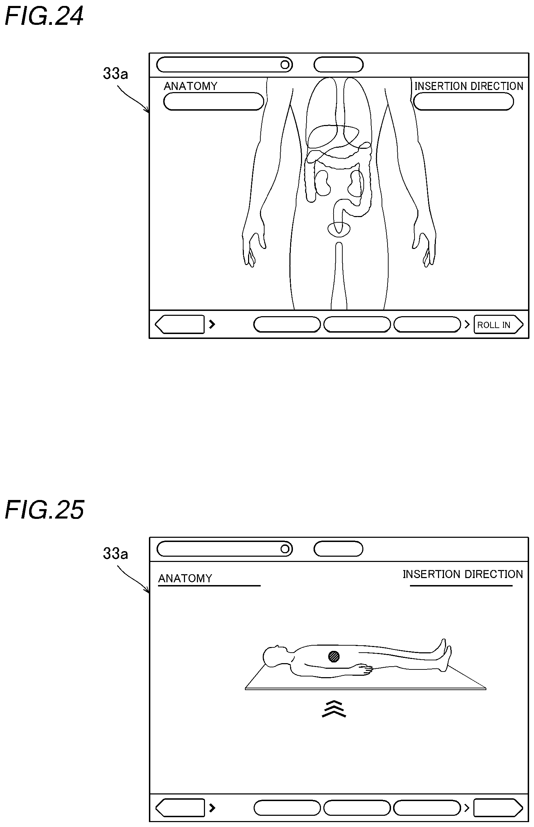

As shown in FIG. 23, in step S1, preparations for positioning the surgical robot 1 are performed. Specifically, as shown in FIG. 24, on a touch panel of the display 33a, a surgical site such as an abdomen and a direction in which the surgical robot 1 is inserted into the patient P are selected.

Then, in step S2, as shown in FIG. 24, a roll-in button displayed on the display 33a is pressed. Thus, a roll-in mode is set, and the movement operation of the arm base 50 and the robot arms 60 is controlled such that the surgical robot 1 takes the roll-in posture. The roll-in posture refers to a posture in which each of the robot arms 60 is folded so as not to interfere with the patient P when the robot arms 60 are positioned above the patient P by movement of the surgical robot 1, in which the arm base 50 is arranged by the positioner 40 such that an imager 51 provided on the arm base 50 can image a region vertically therebelow, and in which the arm base 50 is arranged by the positioner 40 such that the arrangement direction of each of the robot arms 60 corresponds to the surgical site and the insertion direction based on information on the surgical site selected in step S1 and information on the insertion direction selected in step S1. That is, after the roll-in button displayed on the display 33a is pressed such that a transition to the roll-in mode is made, when the joystick 33b is operated while the enable switch 33c is pressed to enable movement of the positioner 40, the control device 130 moves the positioner 40 and each of the robot arms 60 such that the surgical robot 1 automatically takes the roll-in posture.

In this embodiment, an operation of the operator is received to adjust the variable control parameter h before transition of the surgical robot 1 from the current posture to the first posture is completed. For example, when the operator tilts the joystick 33b in the Ya1 direction while the surgical robot 1 is in the current posture of s = 0, the value of the variable control parameter h is increased. Then, the operator tilts the joystick 33b in the Xa1 direction such that the surgical robot 1 transitions from the current posture to the roll-in posture. Consequently, after transition to the roll-in posture, the height H of the surgical robot 1 is higher as compared with a case in which the first posture is not adjusted.

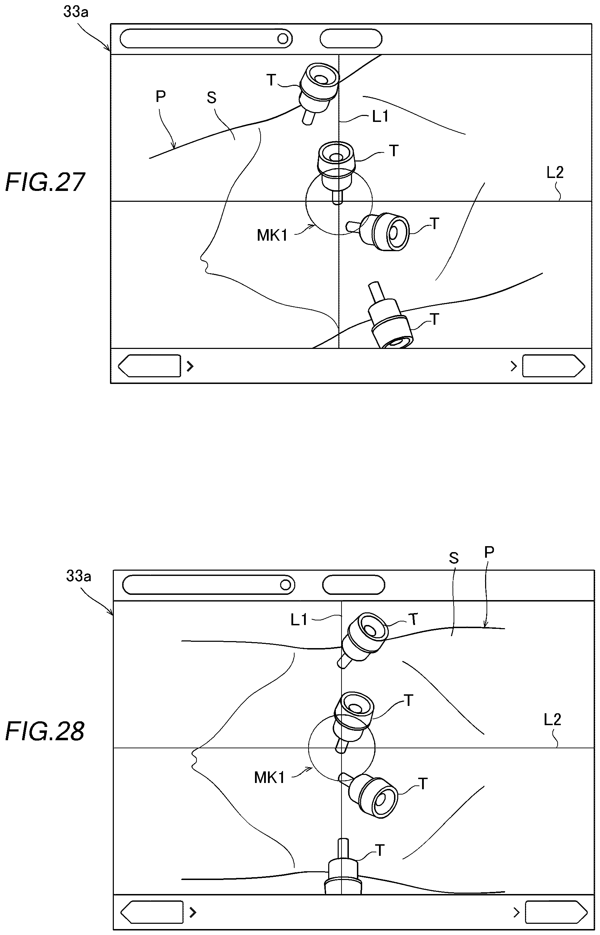

Then, in step S3, as shown in FIG. 25, after the movement operation of the robot arms 60, a screen of the display 33a is switched to an image captured by the imager 51. Then, as shown in FIG. 25, the operator moves the medical cart 3 closer to the surgical table 5 such that the imager 51 provided on the arm base 50 images the surgical table 5 and the patient P placed on the surgical table 5. Specifically, the operator such as a nurse or a technician operates the operation handle 34 while viewing the image displayed on the display 33a to move the medical cart 3 to the vicinity of the patient P. Thus, the trocars T are placed directly below the imager 51. Then, as shown in FIG. 27, the trocar T is placed inside a substantially circular first mark MK1 on the display 33a. Furthermore, as shown in FIG. 28, the operator operates the positioner 40 using the joystick 33b such that the remaining trocars T are placed on a first line L1 or a second line L2 of a second mark MK2 while one trocar T is placed inside the substantially circular first mark MK1 on the display 33a.

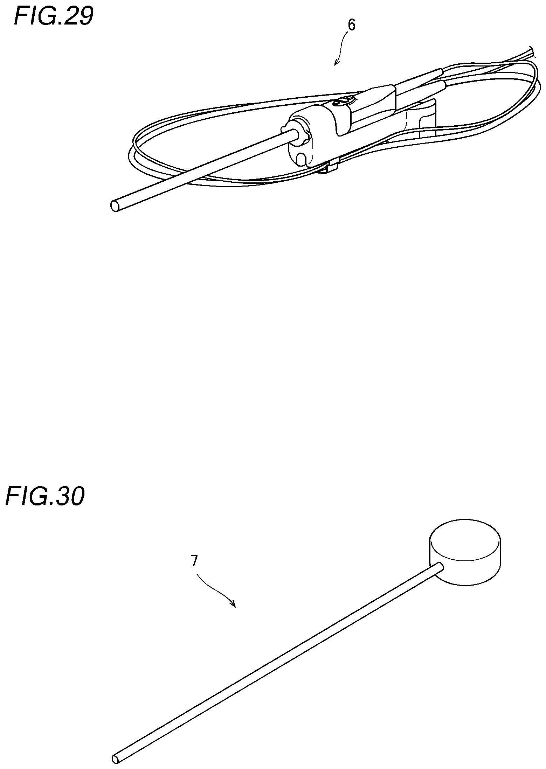

Then, in step S4, the control device 130 transitions the plurality of robot arms 60 to setup postures based on an arm preparation button on the display 33a being pressed. The setup posture is different from the roll-in posture in which each robot arm 60 is folded, and refers to a posture in which the distance between the robot arms 60 is increased such that the endoscope 6 shown in FIG. 29 or the pivot position teaching instrument 7 shown in FIG. 30 can be easily attached to each of the plurality of robot arms 60. The pivot position teaching instrument 7 is an instrument that is attached to the distal end of the robot arm 60 to which the instrument is to be attached, instead of the instrument, when the pivot position PP is taught. Thereafter, the operator attaches the endoscope 6 or the pivot position teaching instrument 7 to each of the plurality of robot arms 60.

Then, in step S5, when the pivot button 85 is pressed in a state in which the distal end of the endoscope 6 or the pivot position teaching instrument 7 attached to the distal end side of the robot arm 60 has been moved to a position corresponding to the insertion position of the trocar T inserted into the body surface S of the patient P by operating the robot arm 60 using the arm operation unit 80, the control device 130 causes the storage 32 to store the pivot position PP. The pivot position PP is stored as one coordinate point, and in pivot position PP setting, the direction of the surgical instrument 4 is not set.

### Advantages of This Embodiment

The joystick 33b is provided to receive an operation of the operator to adjust the variable control parameter h to adjust the first posture before transition of the surgical robot 1 from the current posture to the first posture is completed. The control device 130 is configured or programmed to control the surgical robot 1 to transition from the current posture to the first posture obtained after adjustment of the variable control parameter h. Accordingly, when the surgical robot 1 transitions to the first posture, the first posture after transition can be adjusted before the transition is completed. Therefore, the operator can transition the surgical robot 1 to a posture appropriate for the situation.

The plurality of control parameters include a fixed control parameter. Accordingly, the posture of the surgical robot 1 can be varied from a reference posture defined by the fixed control parameter by changing the variable control parameter h.

The control device 130 is configured or programmed to perform a control to change at least one of the rotation angles of the joints 43 of the positioner 40 and the joints 64 of the robot arm 60 corresponding to control parameters, or the coordinates of the surgical robot 1 corresponding to control parameters based on the adjustment operation received by the joystick 33b. Accordingly, the first posture can be easily adjusted by changing at least one of the rotation angles of the joints 43 of the positioner 40 and the joints 64 of the robot arm 60, or the coordinates of the surgical robot 1.

The joystick 33b is operable to receive an operation of the operator to adjust the variable control parameter h at any time point from the start of transition to the completion of the transition when the surgical robot 1 transitions from the current posture to the first posture. Accordingly, an operation can be received to adjust the variable control parameter h at any time point from the start of the transition to the completion of the transition, and thus the operator can adjust the variable control parameter h in real time while viewing the state of the transition of the posture of the surgical robot 1.

The control device 130 is configured or programmed to control the robot arms 60 and the positioner 40 to transition from the current posture to the first posture obtained after adjustment of the variable control parameter h. Accordingly, the operator can adjust the posture of the surgical robot 1 including the robot arms 60 and the positioner 40.

The joystick 33b is operable to receive an operation of the operator to adjust the control parameter when the surgical robot 1 starts to transition from the current posture to the first posture. Accordingly, the operator can adjust the variable control parameter h at the start of the transition at which the surgical robot 1 is stationary, and thus the operator can easily adjust the variable control parameter h.

The first posture includes at least one of the storage posture in which the surgical robot 1 is stored, the draping posture in which the surgical robot 1 is covered with a sterile drape, the standby posture in which the surgical robot 1 is kept waiting before the start of surgery, the roll-in posture in which the surgical robot 1 is moved to the patient P, the cleaning posture in which the surgical robot 1 is cleaned, or the transportation posture in which the surgical robot 1 is transported. Accordingly, the operator can adjust at least one of the storage posture, the draping posture, the standby posture, the roll-in posture, the cleaning posture, or the transportation posture according to the situation.

The joystick 33b is operable to receive an operation to adjust at least one of the height H of the surgical robot 1 in the first posture, the amount of forward protrusion of the surgical robot 1 in the first posture, the amount of right-left protrusion of the surgical robot 1 in the first posture, or the right-left rotation angle of the surgical robot 1 in the first posture. Accordingly, the operator can adjust at least one of the height H, the amount of protrusion, or the rotation angle of the surgical robot 1.

The robotic surgical system 100 is operable to receive an operation to adjust the variable control parameter h when the operator tilts the joystick 33b in the Ya direction, and the control device 130 is configured or programmed to control the surgical robot 1 to transition from the current posture to the first posture obtained after adjustment of the variable control parameter h when the operator tilts the joystick 33b in the Xa1 direction intersecting with the Ya direction. Accordingly, an operation to adjust the variable control parameter h and an operation to transition to the first posture can be performed using the single joystick 33b. Therefore, the configuration of the robotic surgical system 100 can be simplified as compared with a case in which the operation to adjust the variable control parameter h and the operation to transition to the first posture are performed using separate operation units.

The joystick 33b is operable to receive an operation to adjust the variable control parameter h according to the magnitude of the tilt in the Ya direction. Accordingly, the operator can easily change the adjustment amount of the variable control parameter h by simply changing the amount of tilt of the joystick 33b.

The robotic surgical system 100 is operable to receive an operation to adjust the variable control parameter h when the operator tilts the joystick 33b in the Ya direction while transition of the posture of the surgical robot 1 is enabled by the enable switch 33c, and the control device 130 is configured or programmed to control the surgical robot 1 to transition from the current posture to the first posture obtained after adjustment of the variable control parameter h when the operator tilts the joystick 33b in the Xa1 direction. Accordingly, the enable switch 33c can reduce or prevent that possibility that adjustment of the variable control parameter h is received in a state not intended by the operator.

### Modified Examples

The embodiment disclosed this time must be considered as illustrative in all points and not restrictive. The scope of the present disclosure is not shown by the above description of the embodiment but by the scope of claims for patent, and all modifications or modified examples within the meaning and scope equivalent to the scope of claims for patent are further included.

While the example in which the joystick 33b receives an operation of the operator to adjust the variable control parameter h at any time point from the start of transition of the posture of the surgical robot 1 to the completion of the transition has been shown in the aforementioned embodiment, the present disclosure is not limited to this. For example, the joystick 33b may receive an operation to adjust the variable control parameter h only within a predetermined period of time from the start of the transition of the posture of the surgical robot 1 to the completion of the transition.

While the example in which the first posture of the surgical robot 1 including the robot arms 60 and the positioner 40 is adjusted has been shown in the aforementioned embodiment, the present disclosure is not limited to this. For example, only the postures of the robot arms 60 or only the posture of the positioner 40 may be adjusted.

While the example in which the storage posture, the draping posture, the standby posture, the roll-in posture, the cleaning posture, and the transportation posture are adjusted has been shown in the aforementioned embodiment, the present disclosure is not limited to this. For example, only some of the storage posture, the draping posture, the standby posture, the roll-in posture, the cleaning posture, and the transportation posture may be adjusted. Alternatively, the posture of the surgical robot 1 other than the above postures may be adjusted.

While the example in which the variable control parameter h is changed in a range from 0 to 1 has been shown in the aforementioned embodiment, the present disclosure is not limited to this. For example, the variable control parameter h may be changed in a range other than from 0 to 1.

While the example in which the only control parameter that can be changed by operating the joystick 33b is h has been shown in the aforementioned embodiment, the present disclosure is not limited to this. There may be a plurality of control parameters that can be changed by operating the joystick 33b.

While the example in which when the joystick 33b is tilted in the Ya direction, an operation is received to adjust the variable control parameter h has been shown in the aforementioned embodiment, the present disclosure is not limited to this. For example, an operation may be received to adjust the variable control parameter h when the joystick 33b is rotated about an axis along the Z direction.

While the example in which an operation is received to adjust the variable control parameter h when the joystick 33b is operated has been shown in the aforementioned embodiment, the present disclosure is not limited to this. For example, an operation may be received to adjust the variable control parameter h when a push button, a throttle, an input from a liquid crystal panel, a slide switch, a mouse, a remote control, or the like is operated.

While the example in which the control device 130 controls the surgical robot 1 to transition to the first posture obtained after adjustment of the variable control parameter h has been shown in the aforementioned embodiment, the present disclosure is not limited to this. For example, the first posture obtained after the operator adjusts the variable control parameter h may be registered in the storage 32. When the operator presses a button on the display 33a to register the first posture, the first posture obtained after adjustment of the variable control parameter h is registered in the storage 32. When the first posture registered in the storage 32 is selected by the operator, the control device 130 controls the surgical robot 1 to transition from the current posture to the first posture registered in the storage 32. Accordingly, the first posture obtained after adjustment of the variable control parameter h is registered, and thus when transition to the first posture for the second or subsequent times is made, the surgical robot 1 can transition to the first posture obtained after adjustment of the variable control parameter h without the operator operating the joystick 33b. Consequently, the burden on the operator can be reduced when transition to the first posture for the second or subsequent times is made.

While the example in which the control device 130 controls the surgical robot 1 to transition to the first posture obtained after adjustment of the variable control parameter h has been shown in the aforementioned embodiment, the present disclosure is not limited to this. For example, when transition to the first posture obtained after adjustment of the variable control parameter h is made, the control device 130 may transition the surgical robot 1 to the first posture within a range that does not exceed the limit values of the axial speeds of the joints 43 of the positioner 40 and the joints 64 of the robot arms 60. Specifically, when transition to the first posture obtained after adjustment of the variable control parameter h is made, the control device 130 performs scaling on translational and rotational components of the surgical robot 1 and performs inverse kinematic calculations on the translational and rotational components after scaling to calculate the rotation angles of the joints 43 of the positioner 40 and the joints 64 of the robot arms 60. The term "scaling" refers to adjusting the amount of movement of the surgical robot 1 such that the amount of movement of the surgical robot 1 is smaller than the amount of movement of the joystick 33b by the operator.

While the example in which four robot arms 60 are provided has been shown in the aforementioned embodiment, the present disclosure is not limited to this. In the present disclosure, the number of robot arms 60 may be any other number as long as at least one robot arm is provided.

While the example in which each of the arm portion 61 and the positioner 40 includes a 7-axis articulated robot has been shown in the aforementioned embodiment, the present disclosure is not limited to this. For example, each of the arm portion 61 and the positioner 40 may include an articulated robot having an axis configuration other than the 7-axis articulated robot. The axis configuration other than the 7-axis articulated robot includes six axes or eight axes, for example.

While the example in which the surgical robot 1 includes the medical cart 3, the positioner 40, and the arm base 50 has been shown in the aforementioned embodiment, the present disclosure is not limited to this. For example, the surgical robot 1 may not include the medical cart 3, the positioner 40, or the arm base 50, but may include only the robot arms 60.

The functionality of the elements disclosed herein may be implemented using circuitry or processing circuitry that includes general purpose processors, special purpose processors, integrated circuits, application specific integrated circuits (ASICs), conventional circuitry and/or combinations thereof that are configured or programmed to perform the disclosed functionality. Processors are considered processing circuitry or circuitry as they include transistors and other circuitry therein. In the present disclosure, the circuitry, units, or means are hardware that carries out the recited functionality or hardware that is programmed to perform the recited functionality. The hardware may be hardware disclosed herein or other known hardware that is programmed or configured to carry out the recited functionality. When the hardware is a processor that may be considered a type of circuitry, the circuitry, means, or units are a combination of hardware and software, and the software is used to configure the hardware and/or processor.

### Aspects

It will be appreciated by those skilled in the art that the exemplary embodiments described above are specific examples of the following aspects.

### (Item 1)

A robotic surgical system comprising:
a robot main body to which a surgical instrument is attached;
a control device configured or programmed to control the robot main body to transition from a current posture to a first posture; and
an operation unit to receive an operation of an operator to adjust the first posture before transition of the robot main body from the current posture to the first posture is completed; wherein
the first posture is defined by a plurality of control parameters including a variable control parameter;
the variable control parameter is variable within a predetermined range by operating the operation unit; and
the control device is configured or programmed to control the robot main body to transition from the current posture to the first posture obtained after adjustment of the variable control parameter, based on an operation on the operation unit.

### (Item 2)

The robotic surgical system according to item 1, wherein the plurality of control parameters include a fixed control parameter.

### (Item 3)

The robotic surgical system according to item 2, wherein
the robot main body includes a joint; and
the control device is configured or programmed to perform a control to change at least one of a rotation angle of the joint corresponding to one of the plurality of control parameters, or a coordinate of the robot main body corresponding to another one of the plurality of control parameters based on an adjustment operation received by the operation unit.

### (Item 4)

The robotic surgical system according to any one of items 1 to 3, wherein the operation unit is operable to receive an operation of the operator to adjust the variable control parameter at any time point from a start of transition to completion of the transition when the robot main body transitions from the current posture to the first posture.

### (Item 5)

The robotic surgical system according to any one of items 1 to 4, wherein
the robot main body includes:
a robot arm to which the surgical instrument is attached; and
a robot arm mover to move the robot arm; and
the control device is configured or programmed to control the robot arm and the robot arm mover to transition from the current posture to the first posture obtained after the adjustment of the variable control parameter.

### (Item 6)

The robotic surgical system according to any one of items 1 to 5, wherein the operation unit is operable to receive an operation of the operator to adjust the plurality of control parameters when the robot main body starts to transition from the current posture to the first posture.

### (Item 7)

The robotic surgical system according to any one of items 1 to 6, wherein the first posture includes at least one of:
a storage posture in which the robot main body is stored;
a draping posture in which the robot main body is covered with a sterile drape;
a standby posture in which the robot main body is kept waiting before a start of surgery;
a roll-in posture in which the robot main body is moved to a patient;
a cleaning posture in which the robot main body is cleaned; or
a transportation posture in which the robot main body is transported.

### (Item 8)

The robotic surgical system according to any one of items 1 to 7, wherein the operation unit is operable to receive an operation to adjust at least one of:
a height of the robot main body in the first posture;
an amount of forward protrusion of the robot main body in the first posture;
an amount of right-left protrusion of the robot main body in the first posture; or
a right-left rotation angle of the robot main body in the first posture.

### (Item 9)

The robotic surgical system according to any one of items 1 to 8, wherein
the operation unit includes a joystick;
the robotic surgical system is operable to receive an operation to adjust the variable control parameter when the operator tilts the joystick in a first direction; and
the control device is configured or programmed to control the robot main body to transition from the current posture to the first posture obtained after the adjustment of the variable control parameter when the operator tilts the joystick in a second direction intersecting with the first direction.

### (Item 10)

The robotic surgical system according to item 9, wherein the joystick is operable to receive the operation to adjust the variable control parameter according to a magnitude of a tilt in the first direction.

### (Item 11)

The robotic surgical system according to item 9 or 10, further comprising:
an enable switch to enable or disable transition of a posture of the robot main body; wherein
the robotic surgical system is operable to receive the operation to adjust the variable control parameter when the operator tilts the joystick in the first direction while the transition of the posture of the robot main body is enabled by the enable switch; and
the control device is configured or programmed to control the robot main body to transition from the current posture to the first posture obtained after the adjustment of the variable control parameter when the operator tilts the joystick in the second direction.

### (Item 12)

A control method for a robotic surgical system, the control method comprising:
receiving an operation of an operator to adjust a variable control parameter, which is variable within a predetermined range, to adjust a first posture before transition of a robot main body to which a surgical instrument is attached from a current posture to the first posture is completed; and
transitioning the robot main body from the current posture to the first posture obtained after adjustment of the variable control parameter.

## Claims

1. A robotic surgical system comprising:
a robot main body to which a surgical instrument is attached;
a control device configured or programmed to control the robot main body to transition from a current posture to a first posture; and
an operation unit to receive an operation of an operator to adjust the first posture before transition of the robot main body from the current posture to the first posture is completed; wherein
the first posture is defined by a plurality of control parameters including a variable control parameter;
the variable control parameter is variable within a predetermined range by operating the operation unit; and
the control device is configured or programmed to control the robot main body to transition from the current posture to the first posture obtained after adjustment of the variable control parameter, based on an operation on the operation unit.

2. The robotic surgical system according to claim 1, wherein the plurality of control parameters include a fixed control parameter.

3. The robotic surgical system according to claim 2, wherein
the robot main body includes a joint; and
the control device is configured or programmed to perform a control to change at least one of a rotation angle of the joint corresponding to one of the plurality of control parameters, or a coordinate of the robot main body corresponding to another one of the plurality of control parameters based on an adjustment operation received by the operation unit.

4. The robotic surgical system according to claim 1, wherein the operation unit is operable to receive an operation of the operator to adjust the variable control parameter at any time point from a start of transition to completion of the transition when the robot main body transitions from the current posture to the first posture.

5. The robotic surgical system according to claim 1, wherein
the robot main body includes:
a robot arm to which the surgical instrument is attached; and
a robot arm mover to move the robot arm; and
the control device is configured or programmed to control the robot arm and the robot arm mover to transition from the current posture to the first posture obtained after the adjustment of the variable control parameter.

6. The robotic surgical system according to claim 1, wherein the operation unit is operable to receive an operation of the operator to adjust the plurality of control parameters when the robot main body starts to transition from the current posture to the first posture.

7. The robotic surgical system according to claim 1, wherein the first posture includes at least one of:
a storage posture in which the robot main body is stored;
a draping posture in which the robot main body is covered with a sterile drape;
a standby posture in which the robot main body is kept waiting before a start of surgery;
a roll-in posture in which the robot main body is moved to a patient;
a cleaning posture in which the robot main body is cleaned; or
a transportation posture in which the robot main body is transported.

8. The robotic surgical system according to claim 1, wherein the operation unit is operable to receive an operation to adjust at least one of:
a height of the robot main body in the first posture;
an amount of forward protrusion of the robot main body in the first posture;
an amount of right-left protrusion of the robot main body in the first posture; or
a right-left rotation angle of the robot main body in the first posture.

9. The robotic surgical system according to claim 1, wherein
the operation unit includes a joystick;
the robotic surgical system is operable to receive an operation to adjust the variable control parameter when the operator tilts the joystick in a first direction; and
the control device is configured or programmed to control the robot main body to transition from the current posture to the first posture obtained after the adjustment of the variable control parameter when the operator tilts the joystick in a second direction intersecting with the first direction.

10. The robotic surgical system according to claim 9, wherein the joystick is operable to receive the operation to adjust the variable control parameter according to a magnitude of a tilt in the first direction.

11. The robotic surgical system according to claim 9, further comprising:
an enable switch to enable or disable transition of a posture of the robot main body; wherein
the robotic surgical system is operable to receive the operation to adjust the variable control parameter when the operator tilts the joystick in the first direction while the transition of the posture of the robot main body is enabled by the enable switch; and
the control device is configured or programmed to control the robot main body to transition from the current posture to the first posture obtained after the adjustment of the variable control parameter when the operator tilts the joystick in the second direction.

12. A control method for a robotic surgical system, the control method comprising:
receiving an operation of an operator to adjust a variable control parameter, which is variable within a predetermined range, to adjust a first posture before transition of a robot main body to which a surgical instrument is attached from a current posture to the first posture is completed; and
transitioning the robot main body from the current posture to the first posture obtained after adjustment of the variable control parameter.
